# EUROPEAN PATENT SPECIFICATION

(11) **EP 2 567 237 B1**
(45) Date of publication and mention of the grant of the patent: **05.08.2015**
(21) Application number: 11722126.7
(22) Date of filing: 03.05.2011
(51) Int. Cl.: G01N 33/68

(54) **VCJD CONFIRMATORY SCREENING ASSAY**
SCREENINGTEST ZUR BESTÄTIGUNG VON VCJD
TEST DE DÉPISTAGE DE CONFIRMATION DE LA VMCJ (VARIANTE DE LA MALADIE DE CREUTZFELDT-JAKOB)

(30) Priority: 04.05.2010 GB 201007440
(43) Date of publication of application: 13.03.2013
(73) Proprietor: COMMON SERVICES AGENCY, Edinburgh EH12 9EB (GB)
(72) Inventor: JONES, Michael, Edinburgh EH17 7QT (GB)
(74) Representative: Chapman, Paul Gilmour
(86) International application number: PCT/GB2011/000675
(87) International publication number: WO 2011/138578

(56) References cited:
- GRATHWOHL K-U D ET AL: "SENSITIVE ENZYME-LINKED IMMUNOSORBENT ASSAY FOR DETECTION OF PRPSC IN CRUDE TISSUE EXTRACTS FROM SCRAPIE-AFFECTED MICE", JOURNAL OF VIROLOGICAL METHODS, ELSEVIER BV, NL, vol. 64, no. 2, 1 January 1997 (1997-01-01), pages E205-E216, XP000931168, ISSN: 0166-0934, DOI: DOI:10.1016/S0166-0934(97)02197-6
- CAI K ET AL: "Solvent-dependent precipitation of prion protein", BIOCHIMICA ET BIOPHYSICA ACTA. PROTEIN STRUCTURE AND MOLECULAR ENZYMOLOGY, ELSEVIER, AMSTERDAM; NL, vol. 1597, no. 1, 20 May 2002 (2002-05-20) , pages 28-35, XP004354413, ISSN: 0167-4838, DOI: DOI:10.1016/S0167-4838(02)00282-0
- POLYMENIDOU MAGDALINI ET AL: "A short purification process for quantitative isolation of PrPSc from naturally occurring and experimental transmissible spongiform encephalopathies", BMC INFECTIOUS DISEASES, BIOMED CENTRAL, LONDON, GB, vol. 2, no. 1, 8 October 2002 (2002-10-08) , page 23, XP021015596, ISSN: 1471-2334, DOI: DOI:10.1186/1471-2334-2-23 cited in the application
- MICHAEL JONES ET AL: "Human platelets as a substrate source for the in vitro amplification of the abnormal prion protein (PrP Sc ) associated with variant Creutzfeldt-Jakob disease", TRANSFUSION, vol. 49, no. 2, 1 February 2009 (2009-02-01), pages 376-384, XP55002849, ISSN: 0041-1132, DOI: 10.1111/j.1537-2995.2008.01954.x cited in the application

## Description

### Field of the invention

The present invention relates to an improved method for the detection of prion proteins, especially abnormal prion proteins (PrP^{Sc}), in samples of blood, such as plasma. The invention also relates to kits for use in such methods.

### Background of the invention

The infectious agent responsible for variant Creutzfeldt-Jakob disease (vCJD) can be transmitted from person-to-person (secondary disease transmission) by blood transfusion [Turner and Ludlam (2009) Br. J. Haematol. 144: 14-23] and possibly plasma products [Peden, McCardle, et al (2010) Haemophilia 16: 296-304]. Furthermore, affected individuals are infectious during a protracted pre-clinical asymptomatic disease phase. This has raised concerns that a pool of potentially infectious asymptomatic individuals could exists in a blood donor population leading to further cases of secondary disease transmission [Turner and Ludlam (2008) Br. J. Haematol. 144: 14-23]. Introduction of a routine antemortem screening assay capable of detecting infectivity in blood, especially plasma, could help to reduce the risk of secondary disease transmission through blood transfusion, blood products, organ transplantation and surgery.

Prion diseases, including vCJD, are closely associated with the conversion of the normal host cellular prion protein (PrP^{C}) to an abnormal disease-associated form (PrP^{Sc}) [Prusiner (1998) Proc. Natl. Acad. Sci. USA 95: 13363-13383] and it is assumed that between initial infection and the appearance of symptoms that PrP^{Sc} replicates by conversion of the host PrP^{C} [Soto, Saborio, et al (2002) Trends Neurosci. 25: 390-394]. Hence surrogate assays for vCJD infectivity have focused on the detection of PrP^{Sc}, which is considered by some to be the major (if not sole) component of the infectious agent and is the only unambiguous disease marker identified to date [Aguzzi, Heikenwlader, et al (2007) Nat. Rev. Mol. Cell. Biol. 8: 552-561; Grassi, Maillet, et al (2008) Vet. Res. 39: 33].

A blood/plasma screening assay would need to be highly sensitive, since detection of attogram amounts of PrP^{Sc} per millilitre plasma might be required [Peden, Head, et al (2008) Exp. Opin. Med. Diagn. 2: 207-219]. This formidable challenge is further exacerbated by the fact that plasma contains nanogram per millilitre amounts of PrP^{C} [MacGregor, Hope, et al (1999) Vox Sang. 77: 88-96]. A screening assay would therefore need to be able to specifically detect minute amounts of PrP^{Sc} amidst a mass of PrP^{C} or it could lead to more false positive results than detection of true cases, which would not only have a serious impact on the blood supply, but also pose major ethical problems [Turner and Ludlam (2008) Br. J. Haematol. 144: 14-23]. Therefore, ideally two types of assay will be required, the first would be a rapid, high-throughput assay capable of screening all blood donations and the second would be a confirmatory assay to ensure that any positive results from the initial screening assay are indeed true positives.

Although a number of potential screening assays are currently undergoing evaluation, to date the necessary sensitivity and specificity required has yet to be achieved [Turner and Ludlam (2008) Br. J. Haematol. 144: 14-23; Coste, Prowse, et al (2009) Vox Sang. 96: 284-291]. Either more sensitive assays or methods of amplifying any existing PrP^{Sc} to readily detectable levels need to be developed.

Grathwohl et al (J. of Virological Methods., 1997, 64, nos. 2,1 pages [205-216] discloses a method for recovery of PrP^{Sc} from spleen involving salting-out PrP^{Sc} with NaCl (12% w/v) and subsequent immunological detection using ELISA.

Cai et al (Biochimica et Biophysica acta. Protein structure and molecular enzymology (2002) Vol. 1597, No. 1, p28-35) describes methods and means for the precipitation and recovery of PrP^{Sc} from blood involving precipitation using NaCl/EtOH systems.

Polymendiou et al (BMC Infectious diseases, Biomed Central Vol.2, No.1 (2002) p23 provides a NaCl precipitation protocol (using an end-concentration of 10% (w/v) NaCl) for the recovery of PrP^{Sc} from brain tissue samples and detection of the recovered PrP^{Sc}.

Jones et al (Transfusion (2009) 49 No.1, p376-384) discloses methods and means for recovering and detecting PrP^{Sc} from human platelets , wherein the detection involves Protein Misfolding Cyclic Amplification (PMCA) and confirmation dependent immunoassay (CDI).

### Summary of the invention

The present invention is based on the development of a method that allows the efficient amplification and subsequent detection of PrP^{Sc} from a sample of human blood, such as a plasma sample.

A method of recovering and detecting PrP^{Sc} from a sample of blood, such as a plasma sample, the method comprising: adding sodium chloride (NaCl) to said sample, wherein the final concentration of NaCl is between 5 - 20% w/v, in an amount sufficient to facilitate precipitation of PrP^{Sc} from the sample wherein the amount of NaCl is not sufficient to precipitate other factors which may interfere with a subsequent PrP^{Sc} amplification reaction and centrifuging the sample so as to recover said PrP^{Sc} from said sample and further carrying out protein misfolding cyclic amplification (PMCA) on said recovered PrP^{Sc}

Desirably most, such as at least 75%, 80%, 90%, 95% or substantially all of said PrP^{Sc} is recovered from the sample of blood.

The amount of NaCl to be added should be sufficient to cause said PrP^{Sc} to precipitate out of solution. The final concentration of NaCl is in the region of 5 - 20% w/v, preferably 7.5 - 12.5% w/v and ideally 10% w/v, which not only facilitates the recovery of any PrP^{Sc} from a blood sample, such as plasma, but also ensures that any factors that interfere with the subsequent PrP^{Sc} amplification reaction remain in solution.

Typically the precipitate, which forms a pellet upon centrifugation, is washed (as known conventionally in the art) one or more times, using an appropriate "washing solution". A suitable wash-solution may comprise a buffered solution, such as PBS, Tris-HCl containing a detergent such as Sarkosyl. Washing generally comprises resuspending the pellet in a suitable wash-solution and re-centrifugation to pellet the precipitable material, including any PrP^{Sc} once more.

Following the initial NaCl precipitation and the subsequent washing steps the resulting pellet is resuspended in a suitable substrate for the *in vitro* amplification of PrP^{Sc} by Protein Misfolding Cyclic Amplification.

Protein misfolding cyclic amplification (PMCA), which is based on the assumption that prion propagation follows a mechanism of seeded aggregation, is one such amplification method. In the PMCA reaction samples containing minute amounts of PrP^{Sc} are seeded (diluted) into a suitable substrate, containing excess PrP^{C} and other necessary conversion cofactors, and incubated in a cyclic process consisting of alternating steps of incubation and sonication [Saborio, Permanne, et al (2001) Nature 411: 810-813]. It is assumed that during the incubation phase, PrP^{Sc} aggregates, recruit and convert PrP^{C} molecules into PrP^{Sc} resulting in aggregate growth, sonication then breaks the aggregates into smaller subunits each of which in turn act as seeds to recruit and convert further PrP^{C}. It has been shown that the extent of PrP^{Sc} amplification achieved depends on the number of PMCA cycles carried out [Saborio, Permanne, et al (2001) Nature 411: 810-813] and is only limited by the availability/stability of the PrP^{C} and/or the other conversion cofactors present in the substrate [Castilla, Saa, et al (2005) Nat. Med. 11: 982-985].

To overcome this limitation a process termed serial PMCA (sPMCA) has been developed [Bieschke, Weber, et al (2004) Proc. Natl. Acad. Sci, USA 101: 12207-12211; Castilla, Saa, et al (2005) Nat. Med. 11: 982-985], in which following the first round of PMCA an aliquot of the product is seeded into fresh substrate and the PMCA is repeated, an aliquot of the product from this second round of PMCA is further diluted in fresh substrate and again subjected to PMCA and so on allowing indefinite PrP^{Sc} amplification. In animal studies, sPMCA has been used to successfully amplify PrP^{Sc} to readily detectable levels from buffy coat fractions, leukocytes, plasma and whole blood during the clinical disease phase [Castilla, Saa, et al (2005) Nat. Med. 11: 982-985; Murayama, Yoshioka, et al (2007) J. Gen. Virol. 88: 2890-2898; Thorne and Terry (2008) J. Gen. Virol. 89: 3177-3184; Tattum, Jones, et al (2010) Transfusion] and buffy coat fractions during the pre-clinical disease phase [Saa, Castilla, et al (2006) Science 313: 92-94].

Following PMCA or sPMCA, the resulting reaction products need to be screened using suitable methods to detect PrP^{Sc}. Methods of detecting PrP^{Sc} are known in the art and many typically employ limited proteinase K (PK) digestion to distinguish between PrP^{C} and PrP^{Sc}. Such treatment results in the complete digestion of PrP^{C} whilst retaining the PrP^{Sc} proteinase resistant core (PrP^{res}) which can then be detected by suitable methods such as Western blotting and immunoassay [Grassi, Maillet, et al, (2008) Vet. Res. 39: 33]. However, there is now mounting evidence to suggest that proteinase sensitive forms of potentially infectious PrP^{Sc} exist [Yakovleva, Janiak, et al (2004) Transfusion 44: 1700-1705; Safar, Geschwind, et al (2005) Proc. Natl. Acad. Sci. USA 102: 3501-3506; Pastrana, Sajnani, et al (2006) Biochemistry 45: 15710-15717; Cronier, Gros et al (2008) Biochem. J. 416: 297-305]. Given that we do not know the nature (proteinase resistance) of any PrP^{Sc} that might be found in and amplified from blood/plasma, assays capable of distinguishing between PrP^{C} and PrP^{Sc} without the need for proteinase digestion are favoured [Wadsworth, Joiner, et al (2001) Lancet 358: 191-180]. One such assay is the Conformation Dependent Immunoassay (CDI) [Safar, Wille, et al (1998) Nat. Med. 4: 1157-1165; Bellon, Seyfert-Brandt, et al (2003) J. Gen Virol. 84: 1921-1925; Safar, Geschwind, et al (2005) Proc. Natl. Acad. Sci. USA 102: 3501-3506]. This technique relies on the use of an antibody that binds to an epitope that is exposed in native PrP^{C} but buried in native PrP^{Sc}, only becoming exposed following denaturation. Thus by comparing the CDI results obtained for a sample tested both in its native (N) and denatured (D) states it is possible to determine the sample's CDI D/N ratio and a CDI D/N ratio value above a defined cut-off value (typically the mean D/N ratio obtained for known negative controls plus three standard deviations) is indicative for the presence of PrP^{Sc} in that sample.

A major limitation to the application of PMCA to human prion diseases was the reported need to use a substrate prepared from normal brain tissue of the same species as the target to be amplified. However, it has been shown that human platelets were just as efficient as human brain tissue when used as a substrate source for the amplification of vCJD PrP^{Sc} from human brain tissue [Jones, Peden, et al (2007) J. Pathol. 213: 21-26; Jones, Peden, et al (2009) Transfusion 49: 376-384]. Human platelets could therefore provide a readily available, renewable alternative substrate source for use in human PMCA reactions. However, it was observed that the degree of PrP^{Sc} amplification achieved depended on the prion protein gene codon 129 (*PRNP-*129) genotype of both the seed and the substrate [Jones, Peden et al (2009) Transfusion 49: 376-384]. The human prion protein gene (*PRNP*) exists in two major allelic forms encoding either methionine (M) or valine (V) at codon 129. Consequently, individuals can be methionine homozygous (*PRNP*-129MM), methionine/valine heterozygous (PRNP-129MV) or valine homozygous (*PRNP*-129VV). In PMCA experiments using platelet substrates of known *PRNP* codon 129 genotype [Jones, Peden et al (2008) Neuroreport 19: 1783-1786], vCJD PrP^{Sc} (from a *PRNP*-129 MM individual) was only efficiently amplified in a *PRNP*-129MM platelet substrate, whereas, PrP^{Sc} associated with type 2 MV and W sporadic CJD (sCJD MV2 and sCJD W2, used in the absence of vCJD PrP^{Sc} from *PRNP*-129MV and W individuals) was only efficiently amplified in a *PRNP*-129VV substrate. These observations clearly showed that seed/substrate *PRNP*-129 compatibility had a major influence on PMCA amplification efficiency.

To date all clinically confirmed cases of vCJD have occurred in *PRNP*-129MM individuals [Peden, Head, et al (2008) Expert Opin. Med Diagn. 2: 207-219]. However, evidence of possible asymptomatic vCJD infection, based on the detection of PrP^{Sc}, has also been identified in two *PRNP*-129MV [Peden, Head, et al (2004) Lancet 364: 525-529; Peden, McCardle, et al (2010) Haemophilia 16: 296-304] and two *PRNP-*129W individuals [Hilton, Ghani, et al (2004) J. Pathol. 203: 733-739; Ironside, Bishop, et al (2006) Br. Med. J. 332: 1186-1188]. Furthermore, transmission studies using transgenic mice expressing human PrP of the three possible *PRNP*-129 genotypes have shown that whilst the presence of methionine at codon 129 is associated with efficient disease transmission and short disease incubation, all three genotypes are susceptible to infection [Bishop, Hart, et al (2006) Lancet Neurol. 5: 393-398]. Evidently all individuals, regardless of *PRNP*-129 genotype, are at risk.

To avoid *PRNP-129* incompatibility issues in a PMCA based screening assay either all samples tested would need to be PRNP-129 genotyped to ensure seed/substrate compatibility or alternatively PMCA would need to be carried out in both *PRNP*-129MM and PRNP-129 W substrate. Providing seed/substrate *PRNP*-129 genotype compatibility is addressed, following four rounds of sPMCA in platelet substrate, seeded directly with ten-fold serial dilutions of a 10% (w/v) vCJD brain homogenate, a 4-log (10,000-fold) increase in PrP^{Sc} detection sensitivity, as determined by conformation dependent immunoassay (CDI), has been reported [Jones, Peden, et al (2009) Transfusion 49: 376-384]. Thus, in a preferred embodiment sPMCA in combination with CDI may be used for the amplification and subsequent detection of PrP^{Sc} recovered from blood/plasma samples by NaCl precipitation. However, due to the time taken sPMCA/CDI may be more suited for use as a confirmatory assay rather than an initial rapid, high-throughput screening assay.

Preferably detection of amplified PrP^{Sc} is carried out by confirmation dependent immunoassay (CDI).

### Detailed Description

The present invention will now be further described by way of example and with reference to the figures which show:

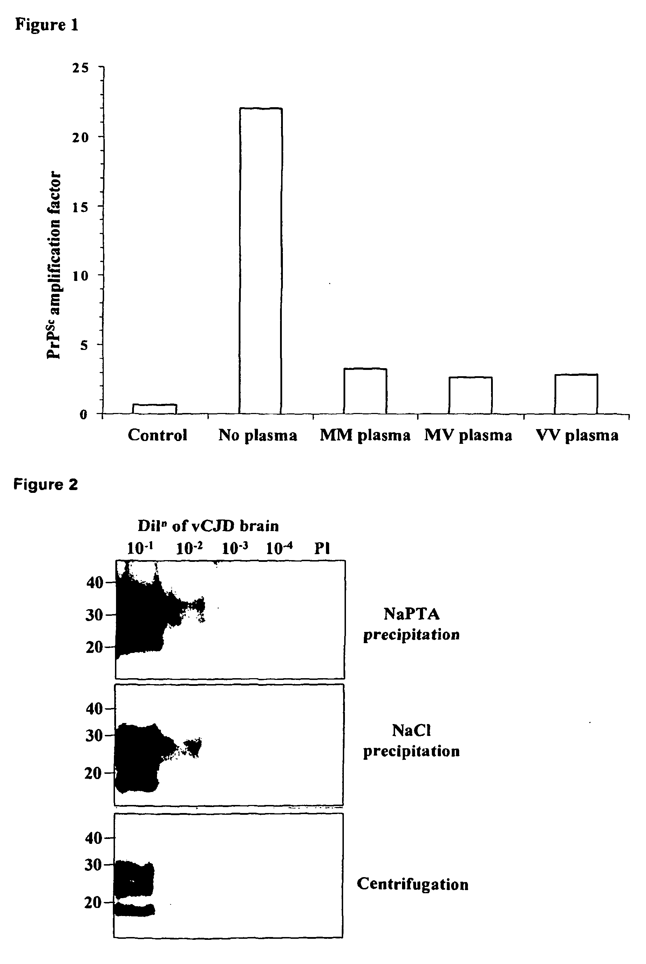

Figure 1 shows the amplification of vCJD PrP^{Sc} directly from spiked plasma
   Plasma (of each *PRNP*-129 genotype, MM plasma, MV plasma, W plasma)) and PMCA conversion buffer (No plasma) spiked with vCJD brain homogenate were seeded directly into platelet substrate. All four seeded reaction mixes and a non-seeded substrate control (Control) were split into two equal lots, with one lot stored at - 80°C (-PMCA) and the other lot subjected to one round of PMCA (+PMCA). Samples - /+ PMCA were screened by CDI with the results obtained expressed as the PrP^{Sc} amplification factor achieved in each seeded substrate. The PrP^{Sc} amplification factor was calculated as the CDI D/N ratio obtained for the +PMCA sample divided by the CDI D/N ratio obtained for the corresponding -PMCA sample.
Figure 2 shows the results from different methods of precipitating/enriching PrP^{Sc} from spiked plasma
   Aliquots of plasma spiked with 10-fold serial dilutions of vCJD brain homogenate were either NaPTA precipitated, NaCl precipitated or subjected to centrifugation alone. The resulting pellets were resuspended, subjected to limited proteinase K digestion and probed for PrP^{res} by Western blotting using mAb 3F4.

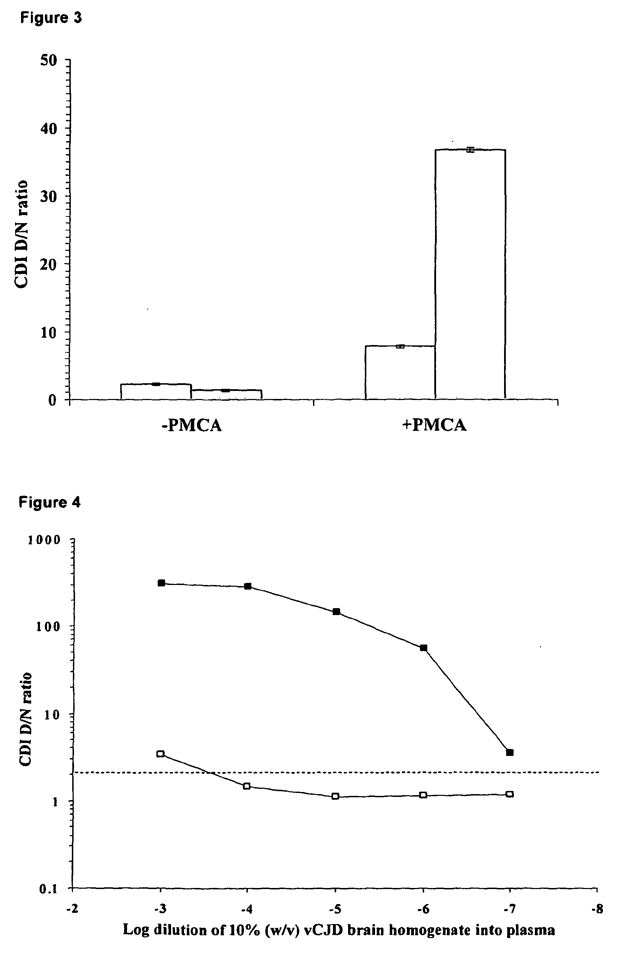

Figure 3 shows the effects on amplification of PrP^{Sc} from the pellet obtained following NaCl precipitation of spiked plasma
   Plasma spiked with vCJD homogenate was NaCl precipitated and the resulting pellet resuspended in the same volume of PMCA conversion buffer as that of the original spiked plasma sample. Aliquots of the original spiked plasma (white bars) and resuspended pellet (grey bars) were seeded into platelet substrate, the seeded samples split into two equal lots with one lot stored at -80°C (-PMCA) and the other lot subjected to one round of PMCA (+PMCA). Samples -/+ PMCA were screened by CDI with the results expressed as the CDI D/N ratio obtained for each sample.
Figure 4 shows the degree of PrP^{Sc} amplification that can be achieved
   Two lots of plasma spiked with 10-fold serial dilutions of vCJD brain homogenate were NaCl precipitated and the resulting pellets were resuspended in platelet substrate. One set of the serial dilutions were stored at -80°C without PMCA (Open squares) and the other set were subjected to four rounds of sPMCA. Following sPMCA, the 4^{th} round PMCA products (Closed squares) where collected and screened alongside the samples without PMCA by CDI with the results expressed as the CDI D/N ratios obtained for each serial dilution. The dashed line represents the CDI cut-off value calculated as the mean CDI D/N ratio obtained for a non-spiked plasma control plus three standard deviations. Any CDI D/N ratio falling above this cut-off would be regarded as positive for PrP^{Sc}.

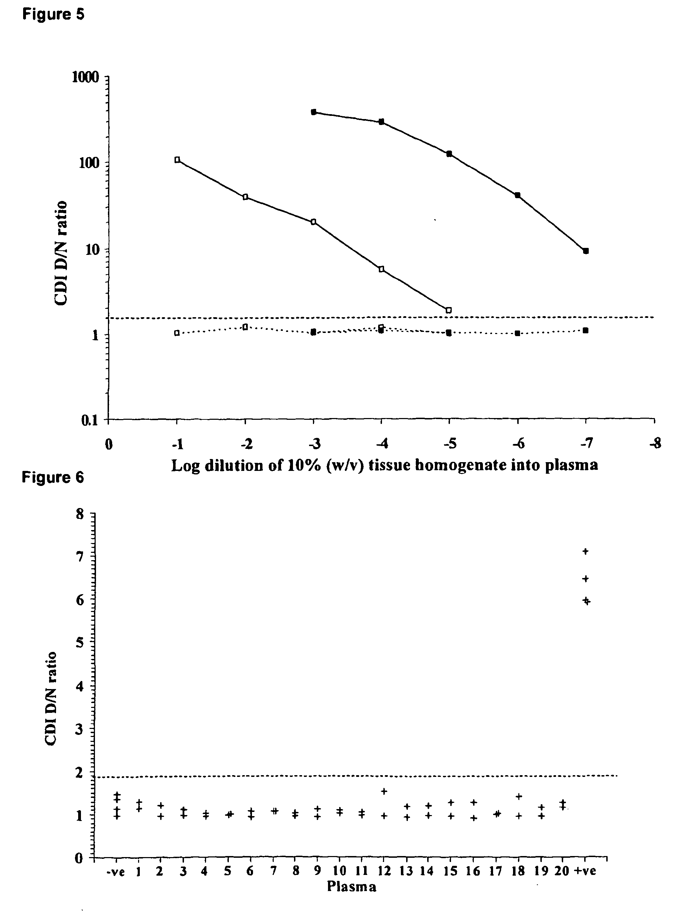

Figure 5 shows the sensitivity and specificity: Amplification of PrP^{Sc} from plasma spiked with vCJD/non-CJD brain and spleen homogenates
   Plasma samples spiked with 10-fold serial dilutions of vCJD brain (Closed squares, solid line); non-CJD brain (Closed squares, dashed line), vCJD spleen (Open squares, solid line) and non-CJD spleen (Open squares, dashed line) homogenates were NaCl precipitated. The resulting pellets were subject to four rounds of sPMCA in platelet substrate and 4^{th} round sPMCA products were screened by CDI with the results obtained expressed as the CDI D/N ratio obtained for each serial dilution. The dashed line represents the CDI cut-off value calculated as the mean CDI D/N ratio obtained for a non-spiked plasma control plus three standard deviations. Any CDI D/N ratio falling above this cut-off would be regarded as positive for PrPSc.
Figure 6 shows the results of screening normal plasma samples
   Duplicate lots of normal plasma, four lots of -ve control plasma and four lots of +ve control plasma (plasma spiked with a 10⁻⁶ dilution of a 10% (w/v) vCJD brain homogenate) were NaCl precipitated. The resulting pellets were resuspended in platelet substrate, subjected to four rounds of sPMCA and the 4^{th} round PMCA products screened by CDI with the results obtained expressed at the CDI D/N ratios obtained. The dashed line represents the CDI cut-off value calculated as the mean CDI D/N ratio obtained for the -ve control plasma samples plus three standard deviations. Any CDI D/N ratio falling above this cut-off would be regarded as positive for PrP^{Sc}.

### Examples

### Example 1

### Preparation of platelet substrate

Platelet lysates for use as PMCA substrates were prepared as previously described [Jones, Peden et al (2009) Transfusion 49: 376-384]. Briefly, buffy coat fractions, obtained from individual donors, were transferred to a 50ml centrifuge tubes. An aliquot (200µl) of each buffy coat was retained for *PRNP*-129 genotyping as previously described [Nurmi, Bishop, et al (2003) Acta Neurol. Scand. 108: 374-378] and the remainder was centrifuged (1050rpm, 20mins). For each buffy coat the resulting platelet rich plasma layer was carefully collected into a pre-weighed 15ml centrifuge tube and centrifuged (4000rpm, 30mins). The plasma was decanted and the platelet pellets were resuspended in 2ml platelet wash buffer (50mM HEPES, 150mM NaCl, 2mM EDTA, 0.09% (w/v) Sucrose, pH 7.4). Following centrifugation (4000rpm, 30mins) the supernatant was discarded, the wet weight of each platelet pellet determined and the pellets stored at -40°C. For preparation of PMCA substrate platelet pellets were lysed in 9 volumes of PMCA conversion buffer (PBS containing 150mM NaCl, 1.0% Triton X-100, 4mM EDTA and Roche Complete EDTA-free® protease inhibitors) to prepare 10% (w/v) lysates. Platelet lysates of the same *PRNP*-129 genotype were pooled, aliquoted and stored at -80°C until used in PMCA reactions.

### Example 2

### Human plasma contains factors, which inhibit the direct amplification of PrP^{Sc}

PMCA conversion buffer (see Example 2) and plasma of each PRNP-129 genotype were spiked with a 10% (w/v) vCJD brain homogenate at a 10⁻³ final dilution. An aliquot (40µl) of each spiked sample was seeded into *PRNP*-129MM platelet substrate (360µl). Duplicate lots (100µl) of each PMCA reaction mix were immediately stored at -80°C (-PMCA). Duplicate lots (100µl) of each PMCA reaction mix were transferred to wells of a thin-walled 96-well PCR plate, the wells sealed, the plate transferred to the microplate horn sonicator [Misonix 3000] containing distilled water (350ml), set up in a 37°C incubator, and incubated for 30 minutes. 48 cycles of PMCA were then carried out with each cycle consisting of a 40 second burst of sonication set at an amplitude setting of 80% (∼300W) followed by incubation at 37°C for 29 minutes and 20 seconds. Following PMCA the PMCA products (+PMCA) were collected. Samples -/+ PMCA were then screened by conformation dependent immunoassay (CDI), modified from the method previously described by Bellon, Seyfert-Brandt, et al (2003) J. Gen Virol. 84: 1921-1925, to determine the relative amounts of PrP^{Sc} in each sample. Briefly, each test sample (100µl) was diluted with PBS/Sarkosyl to a 1 ml final volume containing 2% (w/v) Sarkosyl, Benzonase® (50 units/ml final concentration) and MgCl₂ (1 mM final concentration) were added and the samples incubated for 30min at 37°C. Following the addition of 85µl PrP^{Sc} precipitation solution [4% (w/v) sodium phosphotungstic acid (NaPTA), 34mM MgCl₂, pH7.4 and 0.06% (w/v) amylopectin azure (Sigma)] the samples were incubated overnight at 37°C. After centrifugation (30min, 37°C, 20800g) the resulting NaPTA precipitates were resuspended in 50µl 0.2% (w/v) Sarkosyl in H₂O containing Roche Complete EDTA-free® protease inhibitors. The resuspended precipitates were divided into two equal lots with one lot denatured in 4M guanidine HCl at 81°C for 6min (denatured sample, D) and the other lot left untreated (native sample, N). Both D and N samples were adjusted to a final guanidine concentration of 0.308M in 650µl final volumes and loaded into triplicate wells (200µl/well) of a 96-well black polystyrene plate coated with 0.5µg/well anti-PrP antibody MAR-1 (CSL Behring, Marburg, Germany). Following incubation (2hrs at room temperature with shaking), the wells were washed four times with DELFIA® wash buffer (250µl/well) and blot dried. Europium-conjugated anti-PrP antibody 3F4 (50ng/ml in DELFIA® assay buffer, 200µl/well) was added to all wells and following incubation (2hrs at room temperature with shaking) the wells washed six times with DELFIA® wash buffer (250µl/well) and blot dried. DELFIA® enhancement solution (200µl/well) was added to all wells and following incubation (5min at room temperature with shaking) the time-resolved fluorescence counts for the denatured (D) and native (N) samples were measured using a Victor² fluorometer. The counts obtained for the D samples were divided by the counts obtained for the corresponding N samples to obtain the D/N value for each sample tested. In order to normalise the results obtained for each seed used the results obtained were expressed as the PrP^{Sc} amplification factor achieved by dividing the D/N ratio +PMCA by the corresponding D/N ratio -PMCA. Based on the results obtained (Figure 1) it was evident that whilst efficient amplification of vCJD PrP^{Sc} had been achieved in the substrate seeded with spiked PMCA conversion buffer, little if any amplification had been achieved in substrate seeded with the spiked plasma samples. Evidently plasma, regardless of *PRNP*-129 genotype, contained a factor/factors that inhibited the amplification of PrP^{Sc}. If sPMCA/CDI was to be applied in a confirmatory screening assay to detect PrP^{Sc} in plasma then an initial pre-treatment step to remove these inhibitory factors from plasma would need to be employed. Ideally such a pre-treatment step would also result in the precipitation/enrichment of PrP^{Sc} from the plasma.

### Example 3

### Methods of recovering PrP^{Sc} from spiked plasma

Plasma was spiked with 10-fold serial dilutions (in the range 10⁻¹ to 10⁻⁴) of a 10% (w/v) vCJD brain homogenate. Three methods of precipitating PrP^{Sc} from these spiked plasma samples were then investigated: sodium phosphotungstic acid (NaPTA) precipitation, sodium chloride (NaCl) precipitation and centrifugation alone.

NaPTA precipitation was carried out using a method adapted from that previously described by Bellon, Seyfert-Brandt, et al (2003) J. Gen Virol. 84: 1921-1925. Briefly, each serial dilution (100µl) was diluted with PBS/Sarkosyl to a 1ml final volume containing 2% (w/v) Sarkosyl, Benzonase® (50 units/ml final concentration) and MgCl₂ (1mM final concentration) were added and the samples incubated for 30min at 37°C. Following the addition of 85µl PrP^{Sc} precipitation solution [4% (w/v) sodium phosphotungstic acid (NaPTA), 34mM MgCl₂, pH7.4 and 0.06% (w/v) starch azure] the samples were incubated overnight at 37°C. After centrifugation (30min, 37°C, 20800g) the supernatants were carefully removed and the pellets (visible as blue dots due the starch azure) were stored at -80°C until screened by Western blotting following limited proteinase K digestion using mAb 3F4 to detect PrP^{res} as previously described [22].

For NaCl precipitationwas carried out using a method adapted from the method previously described by Polymenidou, Verghese-Nikalakaki, et al (2002) BMC Infectious Dis. 2: 23. Briefly, each serial dilution (100µl) was diluted in PBS to a 400µl final volume. 20% (w/v) NaCl, 2% (w/v) sarkosyl in PBS (400µl) was added to each sample, the samples incubated at 4°C with shaking (500rpm) in a Thermomixer for 30mins and 1% starch azure (3µl) added to each sample. Following centrifugation (20800g, 30min, 4°C) the supernatants were carefully removed and the pellets (visible as blue dots due the starch azure) were resuspended in 500µl wash buffer (25mM Tris-HCl, 0.05% Sarkosyl, pH8.8). Following centrifugation (20800g, 30min, 4°C) the supernatants were carefully removed and the pellets were stored at -80°C until screened by Western blotting following limited proteinase K digestion using mAb 3F4 to detect PrP^{res} as previously described [22].

For centrifugation alone, each serial dilution (100µl) was simply centrifuged (20800g, 60min, 4°C), the supernatants carefully removed and the pellets were stored at -80°C until screened by Western blotting following limited proteinase K digestion using mAb 3F4 to detect PrP^{res} as previously described [Jones, Peden, et al (2007) J. Pathol. 213: 21-26].

Based on the Western blot results obtained (Figure 2), both NaPTA and NaCl precipitation resulted in similar levels of PrP^{res} recovery, whereas, centrifugation alone resulted in a lower PrP^{res} recovery. It was therefore decided to investigate whether NaCl precipitation not only resulted in the precipitation/enrichment of PrP^{Sc} but also removed the PMCA inhibitory factors previously shown to be present in plasma (see Example 2).

### Example 4

### NaCl treatment not only precipitates PrP^{Sc} from spiked plasma but also allows its amplification in subsequent PMCA reactions

Plasma (200µl) was spiked with a 10% (w/v) vCJD brain homogenate at a 10⁻³ final dilution. An aliquot (20µl) of this spiked plasma was retained to seed subsequent PMCA reactions and the remaining 180µl of spiked plasma was NaCl precipitated as described in Example 3. The resulting pellet was resuspended in 180µl PMCA conversion buffer. *PRNP*-129MM platelet substrate (180µl) was seeded with the original spiked plasma (20µl) and the resuspended pellet post NaCl precipitation (20µl). These reaction mixes were split into 2 X 100µl lots, with one lot stored immediately at - 80°C (-PMCA) and the other lot subjected to 48 cycles of PMCA (+PMCA) as previous described in Example 1. Samples -/+ PMCA were then screened by CDI as previously described in Example 1. Based on the results obtained (Figure 3) whilst there was evidence of some PrP^{Sc} amplification in the reaction seeded directly with spiked plasma, much more efficient PrP^{Sc} amplification was achieved in the reaction seeded with the resuspended pellet obtained following NaCl precipitation. Evidently NaCl precipitation removed the PMCA inhibitory factors present in the plasma allowing the subsequent amplification of PrP^{Sc} from the resuspended pellet fraction.

### Example 5

### A protocol for the precipitation, amplification and subsequent detection of PrP^{Sc} from spiked plasma as a basis for a confirmatory assay for the detection of vCJD infectivity in human plasma.

Control plasma was spiked with 10-fold serial dilutions of a 10% (w/v) vCJD brain homogenate in the range 10⁻³ to 10⁻⁸. Aliquots (2 X 180µl) of each spiked plasma sample and a non-spiked control plasma sample were NaCl precipitated as described in Example 3. The resulting pellets were suspended in *PRNP*-129MM platelet substrate (100µl). One set of samples were immediately stored at -80°C (-PMCA) and the other set and subjected to four rounds of serial PMCA (sPMCA). Briefly, following the first round of PMCA as described in Example 1 aliquots (10µl) of each first round PMCA product were diluted into fresh *PRNP*-129MM platelet substrate (90µl) and a second round of PMCA was carried out. Aliquots (10µl) of each second round PMCA product were diluted into fresh *PRNP*-129MM platelet substrate (90µl) and a third round of PMCA was carried out. Aliquots (10µl) of each third round PMCA product were diluted into fresh *PRNP*-129MM platelet substrate (90µl) and a fourth round of PMCA was carried out and the fourth round PMCA products were collected (+sPMCA). Samples - /+ sPMCA were screened by CDI as described in Example 1. A CDI cut-off value was calculated, mean D/N ratio obtained for the non-spiked control plasma samples plus 3 standard deviations, and any CDI D/N ratio falling above this cut-off was regarded as positive for PrP^{Sc}. From the results obtained (Figure 4) without PMCA (-PMCA) PrP^{Sc} could only be detected in the plasma spiked with the 10⁻³ dilution of the 10% (w/v) vCJD brain homogenate, whereas, following the four rounds of sPMCA (+PMCA) PrP^{Sc} could now be detected in plasma spiked with the 10⁻⁷ dilution of the 10% (w/v) vCJD brain homogenate, this represented a 4-log (10,000-fold) increase in detection sensitivity. Using the protocol outlined in this example (NaCl precipitation, resulting pellet subjected to four rounds of sPMCA in *PRNP*-129MM platelet substrate and subsequent detection of PrP^{Sc} in the 4^{th} round PMCA product) it was possible to detect PrP^{Sc} in plasma spiked with a 10⁻⁷ dilution of a 10% (w/v) vCJD brain homogenate.

### Example 6

### PrP^{Sc} detection sensitivity achieved and its relationship to the detection of vCJD infectivity

Whilst the level of infectivity in the vCJD brain tissue used as the seed source in this study has not been determined, based on previous studies [Bruce, McConnell, et al (2001) Lancet 358: 208-209], infectivity levels in the brain may vary from 10⁵ to 10⁷ ID₅₀/g (ID₅₀ = infectious dose causing disease in 50% of exposed animals). Following four rounds of serial PMCA we were able amplify and detect PrP^{Sc} from a 10⁻⁷ dilution of a 10% (w/v) vCJD brain homogenate spiked into 180µl plasma corresponding to 1.8ng vCJD brain or an ID₅₀ of 1.8 X 10⁻⁴ to 1.8 X 10⁻². Levels of infectivity in the plasma, based on the results obtained from experimental animal models, are between 1-10 ID₅₀/ml in the preclinical and 100 ID₅₀/ml in the clinical disease stage [Peden, Head, et al (2008) Exp. Opin. Med. Diagn. 2: 207-219; Jones, Peden et al (2009) Transfusion 49: 376-384]. Assuming that these figures hold true for levels of vCJD infectivity present in human plasma during the preclinical disease stage, if 180µl of plasma is used to seed the PMCA reaction then the ID₅₀ present would be between 0.18 to 1.8. Based on these estimations (assuming that the detection of PrP^{Sc} correlates with infectivity and that the nature of the infectious agent found in both brain and plasma are the same) then four rounds of serial PMCA should permit the detection of vCJD infectivity in the plasma of individuals with preclinical disease. However, to reduce the risk of disease transmission even further, it would be desirable to detect at least 1ID/whole blood unit which equates to approximately 0.002 ID/ml or 3.6 X10⁻⁴ ID/180µl. Four rounds of sPMCA would just about achieve the sensitivity required to detect 1ID in a unit of whole blood.

### Example 7

### Sensitivity and specificity achieved based on the amplification of PrPSc from plasma spiked with vCJD/non-CJD brain and spleen homogenates

Control plasma was spiked with 10-fold serial dilutions of vCJD and non-CJD 10% (w/v) brain homogenates in the range 10⁻³ to 10⁻⁸ and vCJD and non-CJD 10% w/v spleen homogenates in the range 10⁻¹ to 10⁻⁵. Aliquots (180µl) of each spiked plasma sample and non-spiked control plasma were NaCl precipitated and the resuspended pellets subjected to four rounds of serial sPMCA in PRNP-129MM platelet substrate, as described in Example 5. The fourth round PMCA products were collected and screened by CDI, as described in Example 2, using the mean D/N ratio obtained for the non-spiked plasma samples to determine the CDI cut-off value. From the results obtained (Figure 5) in the PMCA reactions seeded with plasma spiked with vCJD brain and spleen homogenates, PrP^{Sc} was detected down to at least down the 10⁻⁷ dilution of the 10% (w/v) brain homogenate and the 10⁻⁵ dilution of the 10% (w/v) spleen homogenate. No evidence of PrP^{Sc} was detected in the PMCA reactions seeded with plasma spiked with non-CJD brain and spleen homogenates, suggesting that four rounds of sPMCA did not result in the spontaneous/de novo production of PrP^{Sc}. Based on these results it would appear that the protocol used is both highly sensitive and specific for the amplification and subsequent detection of PrP^{Sc} from human plasma.

### Example 8

### Screening normal plasma samples: A further investigation of specificity

Duplicate lots (180µl) of normal plasma samples (n =20), four lots of control plasma and four lots +ve control plasma (control plasma spiked with a 10⁻⁶ dilution of a 10% (w/v) vCJD brain homogenate) were NaCl precipitated, as described in Example 3, and the resulting pellets were resuspended in PRNP-129MM platelet substrate (100µl) and subjected to four rounds of sPMCA as described in Example 5. The fourth round PMCA products were then screened by CDI, as previously described in Example 2, using the CDI D/N ratios obtained for the control plasma to calculate the CDI cut-off value. All PMCA reaction products seeded with the normal plasma samples had CDI D/N ratios below the calculated cut-off (Figure 6) and were therefore classed as negative. All PMCA reaction products seeded with the spiked +ve control plasma had CDI D/N ratios above the calculated cut-off. To further investigate assay specificity a further 250 normal plasma samples are currently being screened in duplication in both MM and W platelet substrates.

### Example 9

### Conclusions

As previously reported [Jones, Peden, et al (2009) Transfusion 49: 376-384] sPMCA/CDI resulted in the efficient amplification and subsequent detection of PrP^{Sc} from vCJD brain homogenate seeded directly into platelet substrate. However, attempts to amplify PrP^{Sc} from plasma spiked with vCJD brain homogenate and seeded directly into platelet substrate failed (Example 2, Figure 1). Evidently plasma, regardless of the *PRNP*-129 genotyope, contained a factor or factors that inhibited the amplification of PrP^{Sc} in subsequent PMCA reactions. If sPMCA/CDI was to be applied, such as in a confirmatory screening assay to detect PrPSc in plasma, then an initial pre-treatment step to remove the inhibitory factors present in plasma would need to be employed. Ideally such a pre-treatment step would also result in the enrichment/precipitation of PrP^{Sc} from the plasma, allowing larger starting volumes of plasma to be used thus potentially increasing the sensitivity of the assay without having to resort to additional rounds of serial PMCA. Three potential methods were investigated NaPTA precipitation, sodium chloride (NaCl) precipitation and centrifugation alone (Example 3, Figure 2). Both NaPTA and NaCl precipitation displayed similar levels of PrPSc recovery, whereas, centrifugation alone resulted in less efficient PrPSc recovery. Based on the inert nature of the NaCl precipitation method, this method of plasma pre-treatment was taken forward for further investigation and it was shown that NaCl precipitation also removed the PMCA inhibitory factors present in plasma allowing subsequent amplification of the precipitated PrPSc (Example 4, Figure 3). A modified sPMCA/CDI protocol was therefore adopted which included the NaCl precipitation plasma pre-treatment step (NaCl/sPMCA/CDI). As outlined in Example 5, duplicate aliquots of plasma spiked with 10-fold serial dilutions of a 10% (w/v) vCJD brain homogenate were NaCl precipitated, the resulting pellet were resuspended directly into platelet substrate. One set of samples was stored at -80°C (-PMCA) and the other set were subjected to four rounds of sPMCA (+PMCA). Samples -/+ PMCA were then screened by CDI. Using the NaCl/sPMCA/CDI protocol a 4-log (10,000-fold) increase in PrP^{Sc} detection sensitivity was achieved allowing detection of PrP^{Sc} from plasma spiked with a 10⁻⁷ dilution of a 10% (w/v) vCJD brain homogenate (Figure 4). As discussed in Example 6, the NaCl/sPMCA/CDI protocol would appear to achieve the necessary detection sensitivity required to detect PrP^{Sc} in plasma from individuals with pre-clinical vCJD. Further studies into the sensitivity and specificity (Example 7) of the NaCl/sPMCA/CDI protocol showed that PrP^{Sc} could be successfully amplified and subsequently detected from plasma spiked with at least a 10⁻⁷ dilution of a 10% (w/v) vCJD brain homogenate and a 10⁻⁵ dilution of a 10% (w/v) vCJD spleen homogenate (Figure 5). No evidence of PrP^{Sc} amplification from plasma spiked with non-CJD brain and spleen homogenates (Example 7, Figure 5) nor from 20 normal plasma samples (Example 8, Figure 6) was observed. Based on the results obtained to date it would appear that the protocol we have developed (NaCl/sPMCA/CDI) can achieve both the necessary sensitivity and specificity needed to detect PrP^{Sc} in the plasma of individuals with pre-clinical vCJD. This protocol could therefore form the basis of an assay, such as a confirmatory vCJD screening assay to ensure the future safety of the blood supply by reducing the risk of further cases of secondary vCJD transmission.

## Claims

1. A method of recovering and detecting PrP^{Sc} from a sample of blood, the method comprising: adding sodium chloride (NaCl) to said sample, wherein the final concentration of NaCl is between 5 - 20% w/v, in an amount sufficient to facilitate precipitation of PrP^{Sc} from the sample wherein the amount of NaCl is not sufficient to precipitate other factors which may interfere with a subsequent PrP^{Sc} amplification reaction and centrifuging the sample so as to recover said PrP^{Sc} from said sample and further carrying out protein misfolding cyclic amplification (PMCA) on said recovered PrP^{Sc}.

2. The method according to claim 1 wherein the sample of blood is a plasma sample.

3. The method according to claim 1 or 2 wherein at least 75%, 80%, 90%, 95% or substantially all of said PrP^{Sc} is recovered from the sample of blood/plasma.

4. The method according to either of claims 1-3 wherein the final concentration of NaCl is between 7.5 - 12.5% w/v.

5. The method according to any preceding claim, further comprising one or more washing steps following the initial NaCl precipitation.

6. The method according to any preceding claim wherein the pellet formed following centrifugation is resuspended in a suitable substrate for the in vitro amplification of PrP^{Sc} by PMCA.

7. The method according to claim 6 wherein the amplification step is carried out using a serial PMCA (sPMCA) process.

8. The method according to any of preceding claims wherein detection of amplified PrP^{Sc} is carried out by conformation dependent immunoassay (CDI).

## Patentansprüche

1. Verfahren zum Rückgewinnen und Ermitteln von PrP^{Sc} aus einer Blutprobe, wobei das Verfahren Folgendes umfasst: Hinzufügen von Natriumchlorid (NaCl) zur Probe, wobei die Endkonzentration von NaCl zwischen 5 und 20% Gew./Vol. liegt, in einer Menge, die ausreicht, um die Ausfällung von PrP^{Sc} aus der Probe zu erleichtern, wobei die Menge von NaCl nicht ausreicht, um andere Faktoren, die eine anschließende PrP^{Sc}-Amplifikationsreaktion beeinträchtigen können, auszufällen und Zentrifugieren der Probe, um das PrP^{Sc} aus der Probe rückzugewinnen und ferner Durchführen einer Protein Misfolding Cyclic Amplification (PMCA) auf dem rückgewonnenen PrP^{Sc}.

2. Verfahren nach Anspruch 1, wobei die Blutprobe eine Plasmaprobe ist.

3. Verfahren nach Anspruch 1 oder 2, wobei mindestens 75%, 80%, 90%, 95% oder im Wesentlichen das gesamte PrP^{Sc} aus der Blut-/Plasmaprobe rückgewonnen wird.

4. Verfahren nach einem der Ansprüche 1 bis 3, wobei die Endkonzentration von NaCl zwischen 7,5 und 12,5 Gew./Vol.% liegt.

5. Verfahren nach einem der vorangehenden Ansprüche, ferner umfassend einen oder mehrere Waschschritte nach der anfänglichen NaCl-Ausfällung.

6. Verfahren nach einem der vorangehenden Ansprüche, wobei der nach der Zentrifugierung gebildete Niederschlag in einem für die In-Vitro-Amplifikation des PrP^{Sc} durch PMCA geeigneten Substrat resuspendiert wird.

7. Verfahren nach Anspruch 6, wobei der Amplifikationsschritt unter Anwendung eines seriellen PMCA-(sPMCA)-Prozesses durchgeführt wird.

8. Verfahren nach einem der vorangehenden Ansprüche, wobei die Ermittlung von amplifiziertem PrP^{Sc} durch konformationsabhängigen Immunoassay (CDI) durchgeführt wird.

## Revendications

1. Procédé de récupération et de détection du PrP^{Sc} à partir d'un échantillon de sang, le procédé comprenant : l'ajout de chlorure de sodium (NaCl) audit échantillon, dans lequel la concentration finale de NaCl se situe entre 5 et 20% en poids/volume, dans une quantité suffisante pour faciliter la précipitation du PrP^{Sc} à partir de l'échantillon dans lequel la quantité de NaCl n'est pas suffisante pour précipiter d'autres facteurs susceptibles d'interférer avec une réaction d'amplification du PrP^{Sc} subséquente et la centrifugation de l'échantillon de manière à récupérer ledit PrP^{Sc} à partir dudit échantillon et, en outre, la réalisation d'une amplification cyclique de pliage anormal de protéine (PMCA) sur ledit PrP^{Sc} récupéré.

2. Procédé selon la revendication 1, dans lequel l'échantillon de sang est un échantillon de plasma.

3. Procédé selon les revendications 1 ou 2, dans lequel au moins 75%, 80%, 90%, 95% ou substantiellement la totalité dudit PrP^{Sc} est récupérée à partir de l'échantillon de sang/plasma.

4. Procédé selon l'une des revendications 1 à 3, dans lequel la concentration finale de NaCl se situe entre 7,5 et 12,5% en poids/volume.

5. Procédé selon l'une quelconque des revendications précédentes, comprenant en outre une ou plusieurs étapes de lavage subséquentes à la précipitation du NaCl initiale.

6. Procédé selon l'une quelconque des revendications précédentes, dans lequel la pastille formée suivant la centrifugation est remise en suspension dans un substrat approprié pour l'amplification in vitro du PrP^{Sc} par PMCA.

7. Procédé selon la revendication 6, dans lequel l'étape d'amplification est effectuée au moyen d'un processus de PMCA (sPMCA) en série.

8. Procédé selon l'une quelconque des revendications précédentes, dans lequel la détection du PrP^{Sc} amplifié est effectuée par immunoessai dépendant de la conformation (CDI).
